(19) Europäisches Patentamt / European Patent Office / Office européen des brevets

(11) **EP 4 003 248 B1**

(12) **FASCICULE DE BREVET EUROPEEN**

(45) Date de publication et mention de la délivrance du brevet:
**30.08.2023 Bulletin 2023/35**

(21) Numéro de dépôt: **20740654.7**

(22) Date de dépôt: **22.07.2020**

(51) Classification Internationale des Brevets (IPC):
**A61F 9/008** *(2006.01)* **A61F 2/14** *(2006.01)*

(52) Classification Coopérative des Brevets (CPC):
**A61F 9/00831; A61F 9/00834; A61L 27/3604; A61L 27/3683;** A61F 2/142; A61F 2009/00872; A61F 2240/001; A61L 2430/16

(86) Numéro de dépôt international:
**PCT/EP2020/070690**

(87) Numéro de publication internationale:
**WO 2021/013892 (28.01.2021 Gazette 2021/04)**

(54) **PROCÉDÉ DE PRODUCTION D'UNE PLURALITÉ D'IMPLANTS À PARTIR D'UNE CORNÉE HUMAINE OU ANIMALE PRÉALABLEMENT PRÉLEVÉE**

VERFAHREN ZUR HERSTELLUNG EINER VIELZAHL VON IMPLANTATEN AUS EINER ZUVOR ENTFERNTEN MENSCHLICHEN ODER TIERISCHEN HORNHAUT

METHOD FOR PRODUCING A PLURALITY OF IMPLANTS FROM A PREVIOUSLY REMOVED HUMAN OR ANIMAL CORNEA

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.07.2019 FR 1908274**

(43) Date de publication de la demande:
**01.06.2022 Bulletin 2022/22**

(73) Titulaires:
 • **Université Jean Monnet Saint-Étienne**
  **42100 Saint Étienne (FR)**
 • **Manutech-USD**
  **42000 Saint Etienne (FR)**
 • **Centre national de la recherche scientifique**
  **75016 Paris (FR)**
 • **Centre Hospitalier Universitaire**
  **42100 Saint Etienne (FR)**

(72) Inventeurs:
 • **GAIN, Philippe**
  **69009 Lyon (FR)**

 • **THURET, Gilles**
  **42330 ST Bonnet les Oules (FR)**
 • **MAUCLAIR, Cyril**
  **49440 Challain la Potherie (FR)**
 • **ALBOURGOL, Samy**
  **42000 Saint Etienne (FR)**
 • **EGAUD, Grégory**
  **42660 Jonzieux (FR)**

(74) Mandataire: **Be IP**
  **Cabinet LTL SAS**
  **Centre d'Entreprise et d'Innovation**
  **56, Bd Niels Bohr**
  **CS 52132**
  **69603 Villeurbanne Cedex (FR)**

(56) Documents cités:
  **WO-A1-2014/140434    US-A1- 2017 027 754**
  **US-A1- 2017 319 329    US-A1- 2019 038 399**

## Description

### DOMAINE DE L'INVENTION

La présente invention concerne le domaine technique général de la production d'implants à partir d'une cornée humaine ou animale.

Plus précisément, l'invention concerne un procédé incluant la découpe d'une cornée pour réaliser une pluralité d'implants pour des applications ophtalmologiques chez l'humain ou l'animal.

### ARRIERE PLAN DE L'INVENTION

La cornée est un élément indispensable à la vision d'un patient : elle constitue en effet la fenêtre par laquelle les images du monde extérieur pénètrent dans l'œil.

La cornée d'un patient peut subir différents dommages - engendrant une perte totale ou partielle de vision ou une menace pour l'intégrité du globe oculaire - liés à différentes affections dont le patient est atteint, telles qu'une ulcération de la cornée de cause infectieuse ou immunitaire et pouvant aboutir à une perforation, un kératocône qui déforme la cornée, une opacité suite notamment à une infection, des néovaisseaux cornéens profonds, ou un oedème cornéen par déficience endothéliale cornéenne.

Lorsque la cornée présente une ulcération proche de la perforation, qu'elle est devenue opaque, déformée ou qu'elle est perforée, le patient est susceptible de bénéficier d'une greffe. Cette greffe peut être totale ou partielle, et de différentes formes.

Par ailleurs, il est également possible de modifier la puissance dioptrique de la cornée en y insérant un implant en forme de lenticule de forme choisie, pour corriger par exemple la presbytie ou une amétropie (myopie ou hypermétropie) en insérant ce lenticule cornéen soit dans une poche réalisée au sein du stroma cornéen soit en surface cornéenne sous l'épithélium de surface. Il est aussi possible de greffer, à la face postérieure de la cornée, une lamelle cornéenne très fine recouverte de cellules endothéliales cultivées en laboratoire pour pallier un déficit en cellules endothéliales (greffe endothéliale par bioingénierie cornéenne, ou *« TEEK »*, acronyme de l'expression anglo-saxonne *« Tissue Engineered Endothélial Keratoplasty »)*.

La greffe partielle de cornée consiste à greffer un fragment de cornée sain issu d'un donneur pour remplacer ou renforcer ou modifier une portion de cornée malade du receveur. Ce fragment de cornée peut combler du tissu manquant (ulcération profonde, perforation), renforcer la cornée (kératocone), modifier la courbure cornéenne (presbytie, amétropies), ou servir de support pour apporter des cellules endothéliales (greffe endothéliale).

Cependant, en raison de dons d'organes très insuffisants et de besoins croissants en cornée, on fait face à une pénurie mondiale de cornées. Pour cette raison, il serait très intéressant de pouvoir optimiser le nombre d'implants réalisés au sein d'une seule et même cornée d'un donneur.

Il a déjà été proposé différents procédés et dispositifs de découpe pour réaliser plusieurs implants en forme de lenticules.

Le document US 2017/319329 décrit notamment un système pourformerdes lenticules cornéens, le système incluant un premier appareil de coupe et un deuxième appareil de coupe. Le premier appareil de coupe incluant un L.A.S.E.R. (acronyme de l'anglais *« Light Amplification by Stimulated Emission of Radiation »*, signifiant *« amplification de la lumière par émission stimulée de rayonnement »* en français) est configuré pour couper une cornée d'un donneur et former une portion de cornée. Plus précisément le premier appareil de coupe est configuré pour couper la cornée du donneur le long d'un axe s'étendant entre une surface antérieure et une surface postérieure de la cornée. Le deuxième appareil de coupe est configuré pour former une pluralité de lenticules à partir de la portion de cornée en formant une série de coupes transversales dans la portion de cornée. La cornée entre deux plans de coupe consécutifs constitue un lenticule. Chaque lenticule est ensuite utilisé pour former un implant cornéen respectif.

Un inconvénient de ce type de dispositif est qu'il ne permet pas de réaliser des plans de coupe de précision. En effet, la portion de cornée peut subir des déplacements antéro-postérieurs, en particulier lors de l'interaction L.A.S.E.R./cornée, préjudiciable à la précision de focalisation du faisceau L.A.S.E.R.

Un autre inconvénient de ce type de dispositif est que la qualité des plans de coupe diminue avec la profondeur. En effet, plus le plan focal est profond dans la cornée, plus l'efficacité du faisceau L.A.S.E.R. diminue. Par ailleurs, le nombre de lenticules réalisable avec ce type de dispositif est limité.

Le document US 2019/0038399 décrit un procédé et un système de découpe d'une cornée permettant de produire une pluralité d'implants dont le profil de surface est conçu pour correspondre d'une manière générale à une forme d'un site d'implantation de l'implant. Le procédé de découpe comprend :

i) le positionnement de la cornée dans un système de blocage incluant des buses d'aspiration pour bloquer la cornée par succion,

ii) la découpe d'une cornée pour obtenir une lamelle, par exemple en utilisant une source LASER femtoseconde ou un microkératome,

iii) la découpe de la lamelle pour obtenir une pluralité de lenticules, par exemple en utilisant une source LASER femtoseconde ou un microkératome,

iv) le façonnage des lenticules en utilisant une source LASER excimère pour obtenir une pluralité d'implants,

v) la répétition des étapes précédentes pour obtenir une pluralité d'implants à partir d'une nouvelle lamelle.

Un inconvénient de la solution décrite dans US 2019/0038399 est qu'elle nécessite la mise en oeuvre de plusieurs étapes de découpe et de façonnage pour obtenir un implant. En effet, le blocage de la cornée par aspiration peut provoquer des modifications ponctuelles des portions de cornée en regard des buses d'aspiration, altérant ainsi la qualité de découpe finale, une fois l'aspiration relâchée et la cornée ayant repris sa forme.

Un autre inconvénient de la solution proposée dans US 2019/0038399 est qu'elle est coûteuse en temps de traitement d'une cornée. En effet, le procédé selon US 2019/0038399 nécessite la mise en oeuvre d'une étape de découpe (en utilisant une source LASER femtoseconde) et d'une étape de façonnage (en utilisant une source LASER excimère) pour chaque lenticule afin d'obtenir un implant utilisable. Par ailleurs, il est nécessaire de mettre en oeuvre les étapes i) à iv) pour chaque nouvelle lamelle découpée.

Un autre inconvénient encore de la solution proposée dans US 2019/0038399 est que la durée de conservation des implants obtenus est limitée dans le temps, et leur stockage peut être difficile à mettre en oeuvre.

Un but de la présente invention est de proposer un procédé de découpe d'une cornée préalablement prélevée permettant de pallier au moins l'un des inconvénients précités.

Plus précisément, un but de la présente invention est de proposer un procédé permettant d'optimiser la découpe d'une cornée afin de réaliser une pluralité d'implants, par exemple en minimisant les déchets de découpe et/ou en maximisant la qualité des implants découpés.

## BREVE DESCRIPTION DE L'INVENTION

A cet effet, l'invention propose un procédé de production d'une pluralité d'implants à partir d'une cornée humaine ou animale préalablement prélevée, ***remarquable en ce que*** le procédé comprend les étapes suivantes :

- déposer la cornée dans un dispositif de maintien incluant des première et deuxième plaques transparentes au faisceau L.A.S.E.R. émis par une source L.A.S.E.R., la cornée étant positionnée entre les première et deuxième plaques pour appliquer une contrainte mécanique sur les faces antérieure et postérieure de la cornée,
- découper, en utilisant le faisceau L.A.S.E.R., la cornée contenue dans le dispositif de maintien pour obtenir une cornée découpée, l'étape de découpe comprenant la génération de bulles de gaz pour former des contours de la pluralité d'implants,
- détacher chaque implant de la cornée découpée,
- dé-cellulariser chaque implant détaché pour obtenir des implants dé-cellularisés,
- lyophiliser chaque implant dé-cellularisé pour obtenir des implants lyophilisés,
- stériliser chaque implant lyophilisé pour obtenir des implants stérilisés,
- emballer chaque implant stérilisé pour obtenir des implants emballés.

Des aspects préférés mais non limitatifs du procédé de découpe selon l'invention sont les suivants :

- les implants peuvent inclure :

  o au moins un implant de renfort incluant une lame circulaire à faces parallèles, et/ou
  o au moins un implant de comblement incluant :

  ▪ une lame circulaire et un téton s'étendant en saillie de l'une des faces de la lame circulaire, ou
  ▪ une rondelle de forme annulaire, et/ou

  ◦ au moins un implant (bi)convexe, et/ou
  ◦ au moins un implant (bi)concave, et/ou
  ◦ au moins un implant servant de support de culture cellulaire et incluant une lamelle circulaire à faces parallèles et d'épaisseur inférieure au implant de renfort ;

- l'étape consistant à déposer la cornée dans un dispositif de maintien peut comprendre les sous-étapes consistant à :

  ◦ positionner le dispositif de maintien sur le chemin optique du faisceau L.A.S.E.R. généré par la source L.A.S.E.R. et orienter le dispositif de maintien de sorte que la première plaque soit plus proche de la source L.A.S.E.R. que la deuxième plaque,
  ◦ émettre le faisceau L.A.S.E.R. généré par la source L.A.S.E.R. au travers de la première plaque pour former des bulles de gaz dans la moitié de l'épaisseur de la cornée la plus proche de la première plaque,
  ◦ retourner le dispositif de maintien de sorte que la deuxième plaque soit plus proche de la source L.A.S.E.R. que la première plaque,
  ◦ émettre le faisceau L.A.S.E.R. généré par la source L.A.S.E.R. au travers de la deuxième plaque pour former des bulles de gaz dans la moitié de l'épaisseur de la cornée la plus proche de la deuxième plaque ;

- le procédé peut également comprendre une étape de détermination de zones de coupe dans la cornée, lesdites zones de coupe correspondant à des surfaces de la cornée au niveau desquelles des bulles de gaz doivent être formées lors de l'étape de découpe ;

- l'étape de détermination peut comprendre les sous-étapes suivantes :

  ◦ acquisition d'une image de la cornée,
  ◦ estimation de l'épaisseur et du diamètre de la cornée,
  ◦ détermination de paramètres de réglage du dispositif de maintien pour appliquer la contrainte mécanique sur la cornée ;

- l'étape de détermination peut comprendre les sous-étapes suivantes :

  ◦ détermination des types d'implant souhaités et de leurs tailles associées,
  ◦ calcul des positions et formes des zones de coupe à réaliser en fonction des types et tailles des implants souhaités, et
  ◦ génération d'un plan de coupe pour minimiser les pertes en tissu cornéen ;

- l'étape de détermination peut comprendre la sous-étape suivante :

  ◦ affichage du plan de coupe, ledit plan de coupe illustrant la cornée et les zones de coupe ;

- l'étape de dé-cellularisation peut comprendre les sous-étapes suivantes consistant à :

  ◦ immerger la cornée reçue dans un liquide de dé-cellularisation, et
  ◦ rincer la cornée avec un liquide de rinçage tel qu'une solution sérum physiologique ;

- l'étape de dépôt de la cornée peut comprendre une sous-étape consistant à appliquer un matériau favorisant la fixation des protéines - tel que la glutaraldéhyde - sur la cornée.

L'invention concerne également un kit chirurgical pour le traitement d'une pathologie oculaire, *remarquable en ce que* le kit comprend au moins un implant obtenu en mettant en oeuvre le procédé décrit ci-dessus.

## BREVE DESCRIPTION DES DESSINS

D'autres avantages et caractéristiques du procédé selon l'invention ressortiront mieux de la description qui va suivre de plusieurs variantes d'exécution, données à titre d'exemples non limitatifs, à partir des dessins annexés sur lesquels :

- La figure 1 est une représentation schématique des étapes d'un procédé de découpe,
- La figure 2 est une représentation schématique des sous-étapes d'une phase de détermination de plans de coupe dans une cornée,

- La figure 3 est une représentation schématique de multiples implants découpés dans une seule cornée,
- La figure 4 est une représentation schématique en vue de dessus de lenticules découpés dans une cornée,
- La figure 5 est une représentation schématique en vue de face d'une variante de réalisation d'un implant *« chapeau »,*
- Le figure 6 est une représentation schématique des sous-étapes d'une phase de dépôt d'une cornée dans un dispositif de maintien,
- La figure 7 est une vue en perspective éclatée du dispositif de maintien d'une cornée,
- La figure 8 est une vue en coupe du dispositif de maintien une fois assemblé,
- La figure 9 est une vue en coupe d'une variante de réalisation du dispositif de maintien une fois assemblé.

## DESCRIPTION DETAILLEE DE L'INVENTION

On va maintenant décrire différents exemples du procédé de découpe d'une cornée pour la préparation d'implants en référence aux figures. Dans ces différentes figures, les éléments équivalents sont désignés par la même référence numérique.

### 1. Généralités

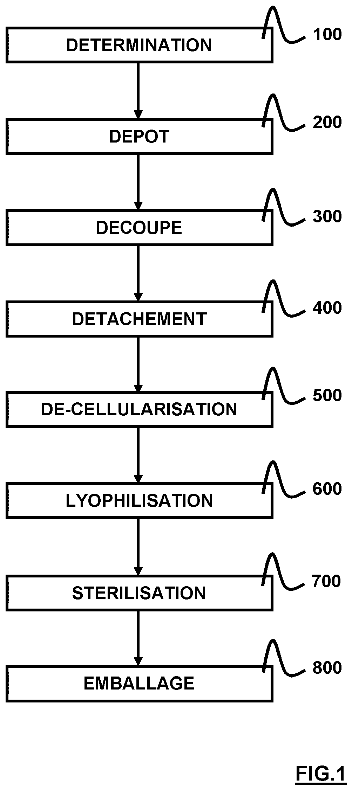

En référence à la figure 1, le procédé de découpe d'une cornée 6 comprend les étapes suivantes :

- Détermination 100 des positions et formes de zones de coupe dans la cornée
- Dépôt 200 de la cornée préalablement prélevée dans un dispositif de maintien de la cornée,
- Découpe 300, en utilisant une source L.A.S.E.R., de la cornée contenu dans le dispositif de maintien pour obtenir une cornée découpée,
- Détachement 400 de chaque implant de la cornée découpée,
- Dé-cellularisation totale (kératocytes, cellules dendritiques et autres cellules immunitaires) 500 de chaque implant préalablement détaché pour obtenir des implants dé-cellularisés,
- Lyophilisation 600 de chaque implant dé-cellularisé pour obtenir des implants lyophilisés,
- Stérilisation 700 de chaque implant lyophilisé pour obtenir des implants stérilisés,
- Emballage 800 dans un dispositif de protection permettant sa réhydratation au bloc, et stockage de chaque implant stérilisé pour obtenir des implants emballés.

## 2. Description détaillée des étapes du procédé de préparation d'un matériau d'allogreffe ou de xénogreffe

### 2.1. Détermination

L'étape de détermination 100 permet de définir la position et la forme des différentes zones de coupe au niveau desquelles la cornée doit être découpé lors de l'étape de découpe 300. Plus précisément, l'étape de détermination 100 permet d'optimiser le nombre de « *pièces* » produites au sein d'une même cornée (sachant que l'épaisseur d'une cornée peut varier entre 100 $\mu$m et 1800 $\mu$m, notamment entre 400 $\mu$m et 1500 $\mu$m, et en particulier entre 500 $\mu$m et 700 $\mu$m dans le cas d'une cornée humaine), et que son diamètre peut varier de 10 à 13 mm dans le cas d'une cornée humaine et de 10 à 20 mm dans le cas d'une cornée animale.

La forme de chaque zone de coupe peut varier en fonction du type d'implant que l'utilisateur souhaite réaliser. En particulier, chaque zone de coupe peut être plane ou incurvée (concave ou convexe), et s'étendre selon une (ou plusieurs) direction(s) sensiblement axiale(s) ou transversale(s) dans la cornée. La combinaison au sein d'une même cornée, de zones de coupe ayant des formes différentes (plane incurvée, cylindrique, etc.) permet de limiter les pertes en tissu cornéen.

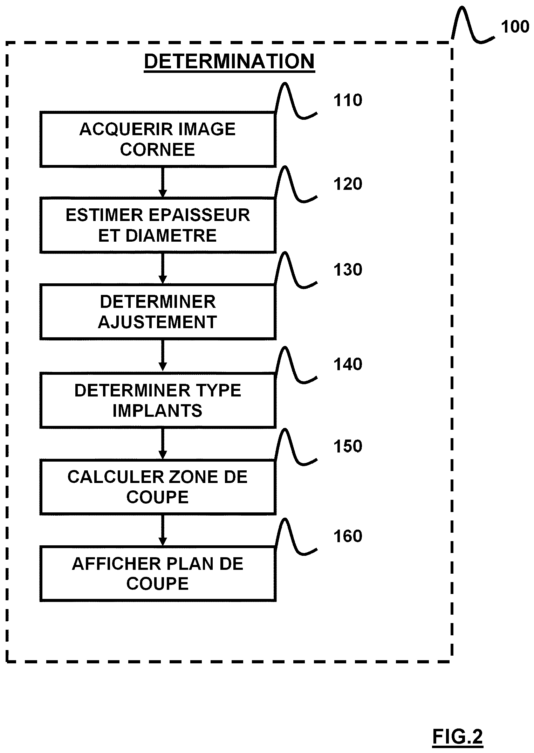

En référence à la figure 2, l'étape de détermination 100 peut comprendre les sous-étapes suivantes :

　i) Acquisition 110 d'une image de la cornée,
　ii) Estimation 120 de l'épaisseur et du diamètre de la cornée,
　iii) Détermination 130 de paramètres de réglage du dispositif de maintien pour appliquer une contrainte mécanique sur la cornée sans l'écraser,
　iv) Détermination 140 des types d'implants souhaités (formes) et de leurs tailles associées,
　v) Calcul 150 des positions et formes des zones de coupe à réaliser en fonction des types et tailles des implants souhaités, et génération d'un plan de coupe pour minimiser les pertes en tissu cornéen,
　vi) Affichage 160 (sur des moyens d'affichage tel qu'un écran) du plan de coupe illustrant le tissu cornéen et les zones de coupe afin de permettre à l'utilisateur de visualiser les zones de coupe,
　vii) Envoi du plan de coupe à des moyens de contrôle d'un dispositif de coupe incluant la source L.A.S.E.R.

La sous-étape d'acquisition 110 d'une image de la cornée à découper peut consister en l'acquisition d'une image de type OCT (signifiant « *Optical Cohérence Tomography* », ou tomographie par cohérence optique), ou de type Scheimpflug (cartographie en lumière visible), ou de type UBM (signifiant « *Ultrasonic Bio Microscopy* », ou biomicroscopie ultrasonore) ou de biomicroscopie (en face ou en fente lumineuse) ou une simple photographie de la cornée. Cette image peut être acquise en utilisant tout système d'acquisition d'image connu de l'homme du métier.

A partir de l'image (ou des images) acquise(s), des moyens de traitement - incluant par exemple un processeur et une mémoire - de l'image (ou des images) acquise(s) estiment 120 l'épaisseur, et le diamètre de la cornée en mettant en oeuvre des techniques de traitement d'image connues de l'homme du métier. Les moyens de traitement peuvent également estimer les courbures antérieures et postérieures de la cornée si la contrainte mécanique appliquée sur la cornée est mise en oeuvre en utilisant un dispositif de maintien incluant des plaques incurvées.

L'épaisseur et le diamètre estimés sont ensuite utilisés par les moyens de traitement pour déterminer 130 des paramètres de réglage du dispositif de maintien. Notamment, dans le cas d'un dispositif de maintien tel que décrit dans la demande de brevet français numéro FR1870835 en date du 17 juillet 2018 (et qui sera présenté plus en détails dans la suite), les moyens de traitement déterminent une distance entre des première et deuxième plaques du dispositif de maintien pour appliquer une contrainte mécanique sur la cornée sans l'écraser.

Par exemple, les moyens de traitement mettent en oeuvre la formule suivante :

$$D_{plaques} = E_{tissu} - Delta,$$

Avec :

- $D_{plaques}$ : la distance séparant les première et deuxième plaques,
- $E_{tissu}$ : l'épaisseur estimée de la cornée,
- Delta : une valeur fixe (par exemple comprise entre 50 et 500 $\mu$m).

Ces paramètres de réglage déterminés sont de préférence affichés sur des moyens d'affichage (tel qu'un écran) pour permettre à l'utilisateur d'ajuster le dispositif de maintien lors de l'étape ultérieure de dépôt 200.

Les moyens de traitement déterminent 140 ensuite les types d'implants souhaités, ainsi que leurs tailles et formes associées. Dans un mode de réalisation de l'invention, les types (et tailles) d'implants souhaités peuvent être renseignés par l'utilisateur à partir de moyens de saisie (tel qu'un clavier). En variante, les types (et tailles) d'implants souhaités peuvent être extraits d'une base de données incluant des types d'implants prédéfinis ainsi que leurs tailles associées. Dans ce cas, les moyens de traitement sélectionnent les types et tailles d'implants de sorte à minimiser les pertes en tissu cornéen.

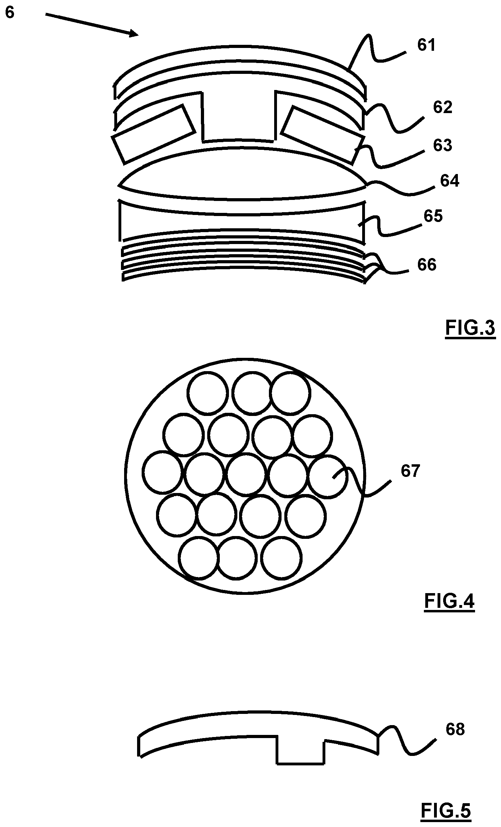

A titre indicatif, la figure 3 illustre différents exemples d'implants 61-66 pouvant être découpés dans une cornée 6. Chaque implant peut par exemple consister en :

- une lame épaisse (par exemple supérieure ou égale

à 100μm) 61 à faces parallèles ; une telle lame peut être utilisée en tant que plan de renfort pour le traitement du kératocône, maladie qui induit un affaiblissement de la biomécanique de la cornée,

- un implant « chapeau » 62 pouvant être utilisé pour boucher une perforation survenue dans une cornée malade, un tel implant ayant la forme générale d'un « chapeau » (allant du chapeau canotier au chapeau haut de forme ) et incluant :

  o une lame circulaire (éventuellement incurvée) de grand diamètre, et
  o un téton cylindrique (ou tronconique) de plus petit diamètre s'étendant en saillie de l'une des faces de la lame circulaire,

  la lame circulaire ayant sensiblement la forme d'un bord annulaire d'un «chapeau», et le téton cylindrique ayant sensiblement la forme d'une calotte plus ou moins élevée allant du « chapeau canotier au chapeau haut de forme »,

- une rondelle 63 de forme annulaire (éventuellement incurvée) ou toroïdale, une telle rondelle pouvant être utilisée pour le comblement d'un ulcère oculaire circonférentiel,

- une lentille biconvexe 64 (ou à plan convexe), dont le diamètre peut par exemple être compris entre 2 (deux) et 9 mm une telle lentille pouvant être utilisée pour le traitement d'une presbytie ou d'une hypermétropie,

- une lentille biconcave 65 (ou à plan concave) dont le diamètre peut par exemple être compris entre 5 et 9 mm, une telle lentille pouvant être utilisée pour le traitement d'une myopie,

- une lamelle 66 à faces parallèles très fine (par exemple comprise entre 40 et 50μm) aptes à constituer un support de culture de cellules endothéliales dans le cadre d'une greffe dite endothéliale.

Bien entendu, d'autres types et formes d'implants peuvent également être découpés dans une cornée 6 en mettant en oeuvre le procédé selon l'invention. Par exemple, en référence à la figure 4, il est possible de découper une pluralité de lenticules 67 de petit diamètre (environ 2 millimètres). Par ailleurs et comme illustré à la figure 5, il est possible de découper un (ou plusieurs) implant(s) « chapeau » à téton excentré 68 (c'est-à-dire un implant « chapeau » dans lequel les axes de révolution du téton et de la lame circulaire ne sont pas confondus), par exemple pour le traitement d'un patient dont l'œil présente une perforation excentrée.

Simultanément ou successivement à l'étape de détermination 140 des types d'implants souhaités, les moyens de traitement calculent 150 les positions et formes des zones de coupe. Cette étape de calcul peut être mise en oeuvre en utilisant toute technique de calcul connue de l'homme du métier.

L'étape de calcul 150 permet de générer un plan de coupe. Ce plan de coupe peut être affiché 160 pour permettre à l'utilisateur de visualiser les zones de coupe et éventuellement de modifier des paramètres d'instructions (ajout d'un éventuel implant et/ou augmentation des dimensions d'un (ou plusieurs) implant(s) pour minimiser les pertes en tissu cornéen).

Si l'utilisateur modifie les paramètres d'instructions, les étapes précédentes iv) à vi) sont réitérées. Sinon, le plan de coupe est transmis à des moyens de contrôle du dispositif de découpe incluant la source L.A.S.E.R.

### 2.2. Dépôt de la cornée

L'étape de dépôt 200 permet de maintenir la cornée en position en vue de sa découpe par la source L.A.S.E.R.

L'étape de dépôt 200 comprend une sous-étape consistant à installer 220 la cornée dans le dispositif de maintien.

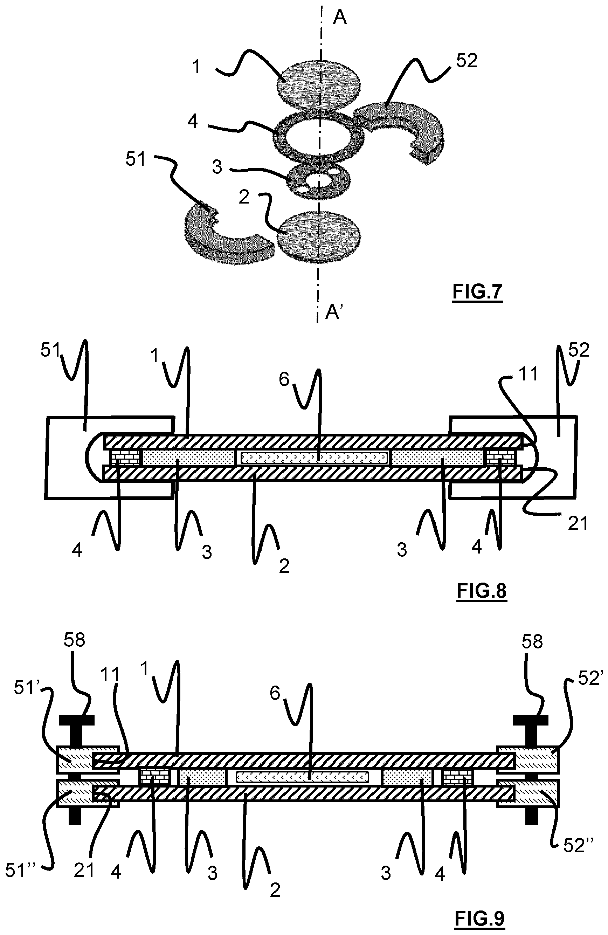

Avantageusement, le dispositif de maintien peut être du type décrit dans la demande de brevet français numéro FR1870835 en date du 17 juillet 2018. En référence à la figure 7, un tel dispositif de maintien (qui sera décrit plus en détails au point 3) comprend :

- un empilement d'éléments incluant :

  ◦ une première plaque 1 transparente au rayonnement électromagnétique,
  ◦ un joint périphérique 4 positionné sur la première plaque 1, le joint périphérique 4 étant destiné à s'étendre autour de la cornée,
  ◦ une deuxième plaque 2 transparente au rayonnement électromagnétique en contact avec le joint périphérique 4,

- et un système de blocage 51, 52 de l'empilement d'éléments apte à plaquer les première et deuxième plaques 1, 2 contre le joint périphérique 4 de sorte à espacer les première et deuxième plaques 1, 2 d'une distance comprise entre 100 μm et 1800 μm, préférentiellement entre 400 μm et 1500 μm, et encore plus préférentiellement entre 500 μm et 700 μm.

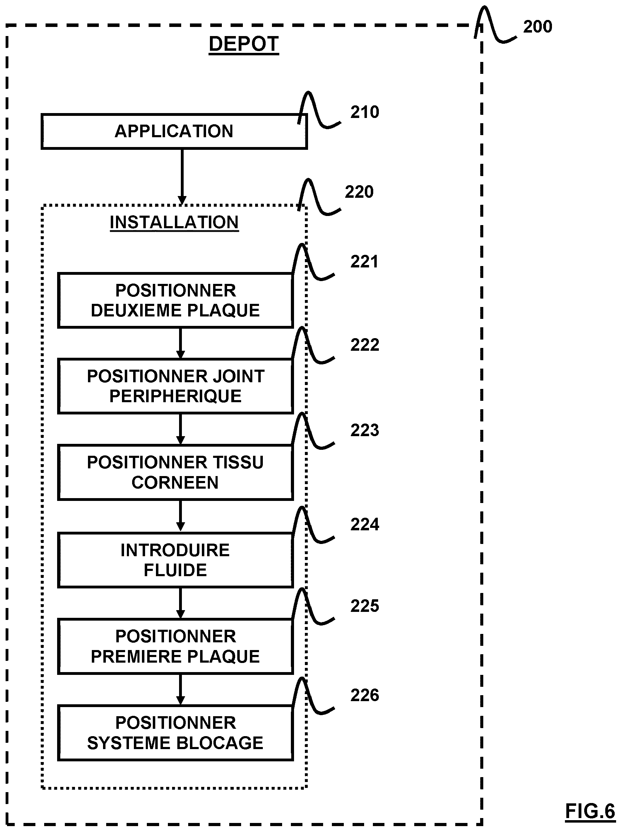

Dans ce cas et comme illustré à la figure 6, la sous-étape consistant à installer 220 la cornée dans le dispositif de maintien décrit dans FR1870835 comporte les opérations suivantes :

- positionner 221 la deuxième plaque 2 sur un support,
- positionner 222 le joint périphérique 4 sur la deuxième plaque 2 pour définir un logement destiné à recevoir la cornée,
- positionner 223 la cornée dans le logement,
- introduire 224 un fluide (liquide ou gel) dans le logement pour parfaire la transmission du rayonnement L.A.S.E.R. généré par la source L.A.S.E.R. lors de l'étape de découpe,

- positionner 225 la première plaque 1 sur le joint 4,
- emmancher 226 en force le système de blocage 51-52 autour des bords latéraux des première et deuxième plaques 1, 2.

Le dispositif de maintien est alors assemblé : la cornée est contrainte entre les première et deuxième plaques 1, 2. Plus précisément, une contrainte mécanique est appliquée sur des faces antérieure et postérieure de la cornée par les première et deuxième plaques 1, 2 du dispositif de maintien selon FR1870835.

Cette contrainte mécanique permet de limiter les risques de déplacement de la cornée lors de l'étape ultérieure de découpe 300.

Cette contrainte mécanique permet également la réalisation de découpes plus fines et plus précises en forçant les bulles de gaz (créée par le faisceau L.A.S.E.R. lors de l'étape de découpe) à s'évacuer de la cornée. La distance entre les zones de coupe adjacentes peut ainsi être diminuée, ce qui permet d'augmenter la quantité d'implants réalisables dans une même cornée.

Par ailleurs, l'application d'une contrainte mécanique sur les deux faces de la cornée permet de contrôler son épaisseur. Il est ainsi possible de réaliser toutes les découpes du plan de coupe, en une seule fois (c'est-à-dire préalablement à la mise en oeuvre de l'étape de détachement 400 des implants) sans intervention multiple à la différence de la solution d'écrite dans US 2019/0038399 qui propose de découper une lamelle, puis de la détacher avant de découper une lamelle suivante (découpe puis détachement puis découpe puis détachement etc.), ce qui multiplie les manipulations.

L'étape de dépôt 200 sur le dispositif de maintien selon FR1870835 permet en outre de réaliser l'étape de découpe (de la cornée) dans des conditions stériles et étanches (vase clos), contrairement à la solution selon US 2019/0038399 dans laquelle le système de blocage par aspiration est ouvert, ce qui ne permet pas de réaliser les opérations de découpes successives dans des conditions stériles.

Dans certaines variantes de réalisation, l'étape de dépôt 200 peut comprendre une sous-étape consistant à appliquer 210 une solution favorisant la fixation des protéines - telle que la glutaraldéhyde - sur la cornée.

Cette sous-étape d'application 210 permet la fixation et la réticulation (ou « *cross-linking* » en anglais, c'est-à-dire le renforcement mécanique par création de ponts covalents entre les protéines collagènes et/ou protéoglycannes composant le stroma) de la cornée afin :

- qu'elle reste fine et transparente,
- qu'elle soit plus facile à découper par application du faisceau L.A.S.E.R. utilisé lors de l'étape de découpe,
- qu'elle soit plus rigide afin de faciliter la mise en oeuvre de l'étape de détachement et que les implants conservent les formes imposées par les zones de coupe, et
- qu'elle résiste durablement aux dégradations enzymatiques après implantation chez le receveur.

Cette sous-étape d'application 210 a également un effet stérilisant vis à vis de certaines bactéries et certains virus.

L'application 210 du matériau favorisant la fixation protéinique peut être mise en oeuvre par toute technique connue de l'homme du métier (immersion de la cornée dans un fluide favorisant la fixation de protéines, projection sur la cornée d'un fluide favorisant la fixation de protéine, etc.).

*2.3. Découpe*

L'étape de découpe 300 permet de générer une pluralité de bulles de gaz dans les zones de coupe afin de faciliter le détachement ultérieur des implants. L'étape de découpe est mise en oeuvre en utilisant un système de découpe incluant une source L.A.S.E.R.

Pour découper la cornée, un faisceau électromagnétique généré par la source L.A.S.E.R. femtoseconde (délivrant des impulsions ultrabrèves et de forte puissance) peut être utilisé.

A chaque impulsion, la source L.A.S.E.R. femtoseconde génère un faisceau. Ce faisceau est focalisé (en un point dit « *de focalisation* ») dans la cornée 6. Une bulle de gaz se forme au point de focalisation, engendrant une disruption très localisée des tissus environnants.

Pour former un plan de découpe dans la cornée 6, une succession de petites bulles de gaz adjacentes sont générées en déplaçant le faisceau. Ainsi, les bulles de gaz sont formées dans la zone de coupe lors de la découpe de la cornée.

Avantageusement, en cas d'utilisation du dispositif de maintien selon FR1870835, l'étape de découpe consiste à appliquer successivement le faisceau électromagnétique généré par la source L.A.S.E.R. par les deux faces de la cornée (i.e. au travers des première et deuxième plaques du dispositif de maintien). En effet, les première et deuxième plaques du dispositif de maintien selon FR1870835 étant transparentes au rayonnement électromagnétique, il est possible de travailler la cornée par les deux faces avec le faisceau L.A.S.E.R..

Il est ainsi possible de limiter la puissance du faisceau L.A.S.E.R. nécessaire lors de la formation des bulles de gaz : la moitié des découpes étant effectuées en passant par une face et la seconde moitié en passant par la face opposée, le faisceau L.A.S.E.R. n'a au maximum que la moitié de la cornée à traverser. L'énergie employée est ainsi plus faible, ce qui limite les risques d'altération de la cornée et augmente la précision de la découpe, lors de la mise en oeuvre de l'étape de découpe et en conséquence améliore la qualité des implants.

### 2.4. *Détachement*

A l'issue de l'étape de découpe, une cornée découpée est obtenue dans laquelle les implants restent solidaires par l'intermédiaire des micro-ponts tissulaires s'étendant entre les pluralités de bulles de gaz générées.

L'étape de détachement 400 consiste à rompre manuellement (ou en utilisant un automate) ces ponts tissulaires pour décrocher les différents implants les uns des autres. Cette opération est mise en oeuvre par l'utilisateur en utilisant des outils chirurgicaux connus de l'homme du métier.

### 2.5. *Dé-cellularisation*

L'étape de dé-cellularisation 500 permet de supprimer les kératocytes et/ou les cellules endothéliales et/ou les cellules épithéliales de chaque implant tout en conservant sa structure et sa conformation. Cette dé-cellularisation permet de diminuer les risques de réaction immunitaire chez le patient greffé. L'homme du métier appréciera que l'étape de dé-cellularisation est mise en oeuvre sur chaque implant. Ceci permet d'améliorer la qualité de la dé-cellularisation, celle-ci étant plus complète sur des portions de cornée (ici les implants peuvent consister en des lamelles fines, etc.) plutôt que sur la cornée entière.

L'étape de dé-cellularisation 500 permet d'obtenir des implants dé-cellularisés présentant ainsi une bonne biocompatibilité et sans en détériorer ni la transparence ni la qualité biomécanique (résistance aux manipulations durant le procédé de fabrication ou ensuite par le chirurgien utilisateur). Sa mise en oeuvre peut être basée sur différentes techniques utilisant des moyens chimiques (utilisation de fluides appropriés à la dé-cellularisation), et/ou des moyens mécaniques (grattage, etc.).

Par exemple, dans un mode de réalisation de l'invention, l'étape de dé-cellularisation 500 peut comprendre les sous-étapes suivantes :

- immerger chaque implant dans un liquide de dé-cellularisation (incluant par exemple du Chlorure de Sodium (NaCl), et/ou de l'Éthylène Diamine Tétra Acétique (EDTA), et/ou un détergent de sodium dodécyl et/ou une enzyme du type ADNase), et
- optionnellement soumettre chaque implant à des vibrations mécaniques et/ou gratter mécaniquement la surface de chaque implant,
- rincer chaque implant avec un liquide de rinçage (tel qu'une solution de sérum physiologique),
- éventuellement répéter les sous-étapes précédentes si l'implant contient encore des cellules.

La mise en oeuvre de l'étape de dé-cellularisation 500 peut être manuelle ou automatique (par exemple en utilisant un automate - tel qu'un automate de changement de bain permettant d'immerger chaque implant (par exemple logé dans un réceptacle à tamis grillagé de type panier métallique ou plastique) dans des bains successifs).

### 2.6. *Lyophilisation*

L'étape de lyophilisation 600 permet de déshydrater les implants. Cette étape de lyophilisation facilite le transport et le stockage ultérieur des implants puisque ceux-ci n'ont plus à être conservés dans un milieu liquide.

Le lecteur appréciera que la lyophilisation diminue la transparence et rigidifie l'implant, mais de façon complètement réversible après réhydratation réalisée au bloc opératoire par le praticien. Ainsi, les qualités biomécaniques (résistance aux manipulations opératoires) ne sont pas altérées une fois l'implant réhydraté.

L'étape de lyophilisation 600 peut être mise en oeuvre à l'aide d'un lyophilisateur, ou en utilisant toute autre technique de lyophilisation connue de l'homme du métier. Une fois le (ou les) implant(s) lyophilisé (s), celui(ceux)-ci est (sont) soumis à une étape de stérilisation.

### 2.7. *Stérilisation*

L'étape de stérilisation 700 permet de réduire la quantité d'organismes nuisibles susceptibles d'être fixés dans ou sur le (ou les) implant(s) lyophilisé (s). L'étape de stérilisation 700 permet en outre d'augmenter la durée de conservation du (ou des) implant(s) lyophilisée(s).

L'étape de stérilisation 700 peut être mise en oeuvre par toute technique de stérilisation connue de l'homme du métier telle que l'irradiation (par exemple soumission de chaque implant lyophilisé à des rayonnements de faisceaux électroniques, des rayonnements gamma ou de la lumière ultraviolette) pendant une certaine période de temps.

### 2.8. *Emballage*

A l'issue de l'étape de stérilisation 700, chaque implant est emballé 800 dans un emballage de conditionnement permettant le stockage stérile et le transport de l'implant. L'emballage permet en outre de préserver l'implant des traumatismes et permet sa réhydratation au bloc opératoire.

A titre indicatif, on va maintenant décrire plus en détails le dispositif de maintien selon FR1870835.

### 3. *Dispositif de maintien*

En référence aux figures 7 à 9, on a illustré un exemple de dispositif de maintien utilisé lors de la mise en oeuvre du procédé de découpe selon l'invention.

Le dispositif comprend :

- des première et deuxième plaques 1, 2 transparentes à un rayonnement électromagnétique,

- éventuellement une entretoise 3 destinée à être positionnée entre les première et deuxième plaques 1, 2,
- un joint périphérique 4 destiné à être positionné entre les première et deuxième plaques, le joint périphérique s'étendant autour de l'entretoise,
- un système de blocage 51-52 pour l'assemblage des plaques 1, 2, de l'entretoise 3 et du joint périphérique 4.

### 3.1. *Plaques transparentes*

Chaque plaque 1, 2 est constituée dans un (ou plusieurs) matériau(x) biocompatible(s), stérilisable(s) et transparent(s) à un rayonnement électromagnétique émis par une source de rayonnement - telle qu'un L.A.S.E.R. ou tout autre type de source de rayonnement connue de l'homme du métier pour le traitement de la cornée.

Dans le mode de réalisation illustré à la figure 7, chaque plaque 1, 2 est réalisée dans un unique matériau, comme du verre ou du poly(méthacrylate de méthyle) ou tout autre matériau connu de l'homme du métier.

Dans certaines variantes de réalisation, chaque plaque 1, 2 peut être composée d'une superposition de couches de matériaux différents. Par exemple, dans une variante de réalisation, chaque plaque 1, 2 est composée d'une couche de matériau rigide (tel que du verre) s'étendant entre deux couches de matériau souple (par exemple à base de silicone) :

- la couche de matériau rigide permet d'augmenter la résistance mécanique de la plaque 1, 2, tandis que
- les couches de matériau souple permettent de limiter les risques de dispersion de morceaux de la couche de matériau rigide en cas de brisure de celle-ci.

Chaque plaque 1, 2 peut également comprendre des renforts pour augmenter sa résistance mécanique. Les renforts s'étendent par exemple au niveau des bords 11, 21 de la plaque 1, 2. Les renforts peuvent consister en des tiges de matériau rigide - tel que du titane ou de l'acier inoxydable ou tout autre métal biocompatible connu de l'homme du métier - intégrées dans la plaque 1, 2.

En variante, les renforts peuvent être dans le même matériau que la plaque 1, 2. Par exemple, les renforts peuvent consister en une (ou plusieurs) zone(s) périphérique(s) de la plaque 1, 2 ayant une (des) épaisseur(s) supérieure(s) à l'épaisseur d'une zone centrale de la plaque 1, 2. Ainsi, la plaque 1, 2 peut comprendre des zones épaisses pour renforcer sa résistance mécanique et des zones amincies pour une meilleure transmission du rayonnement électromagnétique.

Chaque plaque 1, 2 peut s'étendre sensiblement dans un plan ou être concave/convexe, la courbure (ou l'absence de courbure) de chaque plaque 1, 2 dépendant de l'application visée. Dans tous les cas, les première et deuxième plaques sont destinées à s'étendre parallèlement l'une à l'autre. On entend, dans le cadre de la présente invention, par « *plaques planes/concaves/convexes parallèles* », des plaques dont l'espacement est constant en tout point. Ainsi, la distance entre les première et deuxième plaques est constante, et comprise entre 100 $\mu$m et 1800 $\mu$m, préférentiellement entre 400 $\mu$m et 1500 $\mu$m, et encore plus préférentiellement entre 500 $\mu$m et 700 $\mu$m. Ceci permet de contraindre mécaniquement les faces antérieure et postérieure de la cornée 6 pour assurer son blocage en position dans le dispositif de maintien.

Dans le mode de réalisation illustré à la figure 7, chaque plaque 1, 2 est de forme circulaire. Toutefois, il est bien évident pour l'homme du métier que d'autres formes sont envisageables pour chaque plaque 1, 2 (carrée, rectangulaire, triangulaire, etc.).

### 3.2. *Entretoise*

L'entretoise 3 constitue une pièce intermédiaire destinée à être positionnée entre les première et deuxième plaques 1, 2. Elle permet de maintenir une distance prédéfinie entre les première et deuxième plaques 1, 2.

L'entretoise permet également de limiter le déplacement de la cornée dans le plan perpendiculaire à un axe longitudinal A-A' du dispositif.

L'entretoise 3 est préférentiellement rigide. Toutefois, l'entretoise 3 peut également être élastiquement déformable. L'entretoise 3 est par exemple réalisée dans un matériau biocompatible et stérilisable, notamment à base de silicone.

L'entretoise 3 comprend un (ou plusieurs) orifice(s) principal (principaux). L'orifice principal peut être circulaire ou présenter tout autre forme souhaitée (carrée, rectangulaire, etc.). La paroi latérale de chaque orifice principal définit, avec les faces internes des première et deuxième plaques 1, 2, un logement destiné à contenir une cornée 6.

De préférence, le diamètre de l'orifice est sensiblement égal au diamètre de la cornée 6 pour contraindre latéralement celle-ci.

Avantageusement, l'entretoise 3 peut également comprendre un (ou plusieurs) compartiment(s) de dégazage. Ce (ou ces) compartiment(s) de dégazage permet(tent) un stockage des bulles de gaz formées dans la cornée 6 lors de l'application d'un rayonnement électromagnétique en vue de sa découpe.

Chaque compartiment peut être connecté à un (ou plusieurs) orifice(s) principal (principaux) par l'intermédiaire d'un (ou plusieurs) canal (canaux) de connexion. Ce (ou ces) canal (canaux) permet(tent) l'acheminement des bulles de gaz formées dans la cornée 6 vers le compartiment de dégazage.

### 3.3. *Joint périphérique*

Le joint périphérique 4 permet d'assurer l'étanchéité latérale du dispositif une fois les plaques transparentes 1, 2 assemblées, en particulier au niveau des bords 11, 21 des première et deuxième plaques 1, 2.

Avantageusement, le joint périphérique 4 est réalisé dans un matériau élastomère biocompatible et stérilisable, par exemple à base de silicone.

Le joint périphérique 4 peut être de forme annulaire. Toutefois, il est bien évident pour l'homme du métier que le joint périphérique 4 peut présenter d'autres formes (carrée, rectangulaire, triangulaire, etc.), notamment en fonction de la forme des première et deuxième plaques 1, 2.

L'épaisseur du joint périphérique 4 et l'épaisseur de l'entretoise 3 définissent la distance entre les première et deuxième plaques 1, 2, et donc la contrainte mécanique d'épaisseur appliquée sur la cornée 6 par lesdites plaques 1, 2. Des joints 4 et entretoises 3 de plusieurs épaisseurs peuvent être prévus pour adapter l'épaisseur du dispositif à l'épaisseur de la cornée 6, ou à des choix spécifiques de l'utilisateur. Notamment dans un mode de réalisation, l'épaisseur du joint périphérique (et/ou de l'entretoise) peut être comprise entre 100 μm et 1800 μm, préférentiellement entre 400 μm et 1500 μm, et encore plus préférentiellement entre 500 μm et 700 μm. Ceci permet d'espacer les première et deuxième plaques d'une distance garantissant la contrainte mécanique des faces antérieure et postérieure de la cornée 6 afin assurer le blocage en position de la cornée 6 dans le dispositif de maintien.

En variante, le joint 4 et l'entretoise 3 peuvent être réalisés dans une matière expansible et/ou compressible permettant de s'adapter à différentes distances entre les première et deuxième plaques 1, 2. Dans ce cas, le réglage de cette distance est assuré par le système de blocage 51, 52 afin que les première et deuxième plaques soient espacées d'une distance comprise entre 100 μm et 1800 μm, préférentiellement entre 400 μm et 1500 μm, et encore plus préférentiellement entre 500 μm et 700 μm.

### 3.4. Système de blocage

Le système de blocage 51, 52 permet de maintenir en position l'assemblage composé des première et deuxième plaques 1, 2, du joint 4 et de l'éventuelle entretoise 3. Il garantit la stabilité de cet assemblage et permet sa manipulation par l'utilisateur sans risque d'ouverture intempestive.

Le système de blocage 51 ,52 est amovible pour permettre :

- l'insertion de la cornée 6 dans le dispositif préalablement à sa découpe,
- la récupération des implants une fois la cornée 6 découpée.

En référence à la figure 7, le système de blocage peut comprendre un cadre destiné à encercler les bords 11, 21 des première et deuxième plaques 1, 2. Le cadre peut être composé en deux parties, en particulier le cadre peut inclure des première et deuxième portions 51, 52.

Chaque portion 51, 52 consiste par exemple en un demi-cylindre incluant une lumière traversante centrale s'étendant le long de l'axe longitudinal A-A' du dispositif. Plus précisément, chaque portion comprend des plateaux annulaires supérieur et inférieur et une paroi latérale :

- la face interne du plateau supérieur est destinée à venir en regard de la face externe de la première plaque 1,
- la face interne du plateau inférieur est destinée à venir en regard de la face externe de la deuxième plaque 2, et
- la face interne de la paroi latérale est destinée à venir en regard des bords 11, 21 des plaques 1, 2 et de la face externe du joint 4.

Le plateau supérieur et/ou le plateau inférieur de chaque portion 51, 52 peut comprendre un (ou plusieurs) trou(s) pour le passage d'un élément de serrage - tel qu'une vis ayant une tige filetée - permettant d'appliquer une force selon la direction longitudinale A-A' et tendant à plaquer les plaques 1, 2 contre le joint 4 et l'entretoise 3. Cet élément de fixation permet en outre de régler la distance désirée entre les première et deuxième plaques 1, 2 (et donc la contrainte mécanique appliquée sur la cornée 6).

Les première et deuxième portions 51, 52 peuvent également comprendre des moyens de fixation (non représentés) pour permettre la solidarisation desdites portions ensemble.

En variante et comme illustré à la figure 9, le système de blocage peut comprendre des premier et deuxième cadres élémentaires :

- le premier cadre élémentaire comprend des première et deuxième portions élémentaires 51', 52' destinées à encercler le bord 11 de la première plaque 1, et
- le deuxième cadre élémentaire comprend des première et deuxième portions 51", 52" destinées à encercler le bord 21 de la deuxième plaque 2.

Dans ce cas, les premier et deuxième cadres élémentaires sont solidarisés par l'intermédiaire d'une pluralité de vis micrométriques 58 permettant de régler l'écartement entre les premier et deuxième cadre élémentaires, et donc l'écartement entre les première et deuxième plaques 1, 2, de sorte que la distance entre les première et deuxième plaques soit comprise entre 100 μm et 1800 μm, préférentiellement entre 400 μm et 1500 μm, et encore plus préférentiellement entre 500 μm et 700 μm.

#### 4. *Conclusions*

Le procédé de découpe d'une cornée décrit précédemment permet d'optimiser le recyclage des cornées humaines/animales en découpant plusieurs implants dans une même cornée.

Grâce à ce procédé de découpe, il est possible d'obtenir différents types d'implants :

- des implants de comblement par exemple « *en chapeau* » pour réparer des perforations oculaires ou « *en rondelle* » pour réparer des ulcérations circonférentiel les,
- des lames épaisses à faces parallèles pour renforcer la structure de kératocône. Ces lames épaisses peuvent servir aussi à renforcer des ectasies cornéennes secondaires à des chirurgies réfractives, ou des faiblesses de la sclère (post opératoire de décollement de rétine, de plaie), ou encore à recouvrir des drains de valve anti-glaucomateuse extériorisés, etc.
- des lenticules (bi)convexes pour corriger une presbytie ou une hypermétropie,
- des lenticules (bi)concaves pour corriger une myopie.

Les implants obtenus en mettant en oeuvre le procédé de découpe gardent leurs caractéristiques de transparence et de solidité, sans cellules déclenchant une réaction immunitaire. Ils sont stockables à température ambiante, avec une durée de péremption allongée, et sont utilisables immédiatement après une simple réhydratation.

La combinaison du procédé de découpe décrit ci-dessus au dispositif de maintien selon FR1870835 présente en outre de nombreux avantages additionnels :

- le fait que les première et deuxième plaques du dispositif de maintien soient transparentes au rayonnement électromagnétique permet de travailler la cornée par les deux faces (le faisceau L.A.S.E.R. utilisé lors de l'étape de découpe peut être appliqué via la face antérieure et via la face postérieure de la cornée), et donc de réduire la puissance du faisceau L.A.S.E.R. afin de limiter les risques de détérioration des implants lors de la découpe ;
- la contention de la cornée permet :

  o de maintenir la cornée immobile, même lorsque la cornée est soumise à un rayonnement lumineux,
  o d'obtenir une position contrainte connue et stable (il est ainsi possible de retourner le dispositif sans perte des repères pour poursuivre une découpe initiée sur une face et terminée sur l'autre),

- l'association des première et deuxième plaques au joint périphérique permet la préparation de la cornée dans des conditions stériles et étanches (vase clos), et facilite la manipulation de la cornée sans compromettre sa stérilité.

Par ailleurs, l'utilisation d'un dispositif de maintien par contention de la cornée par les deux faces permet de découper une pluralité d'implants en une seule étape, contrairement à la solution décrite dans US 2019/0038399 qui nécessite la découpe d'une lamelle dans la cornée, la dissection de la lamelle, la découpe de lenticules dans la lamelle, le façonnage des lenticules pour obtenir des implants, et la répétition des étapes précédentes pour obtenir des implants à partir d'une nouvelle lamelle. Elle permet en outre d'utiliser une unique source LASER femtoseconde pour réaliser cette étape de découpe, contrairement à la solution décrite dans US 2019/0038399 qui nécessite :

- l'utilisation d'une source LASER femtoseconde (ou d'un dispositif de découpe de type microkératome) pour la découpe d'une lamelle et la découpe des lenticules dans la lamelle, et
- l'utilisation d'une source LASER excimère pour façonner chaque lenticule afin d'obtenir les implants.

Par ailleurs, l'étape de lyophilisation des implants permet d'augmenter la durée de conservation des implants et facilite leur stockage sur étagère.

Le lecteur aura compris que de nombreuses modifications peuvent être apportées à l'invention décrite précédemment sans sortir matériellement des nouveaux enseignements et des avantages décrits ici.

Par exemple, dans la description qui précède, l'étape de détermination est présentée comme étant mise en oeuvre préalablement à l'étape de dépôt de la cornée dans le dispositif de maintien. Il est bien évident pour l'homme du métier que cette étape de détermination peut être mise en oeuvre postérieurement à l'étape de dépôt.

#### Revendications

1. Procédé de production d'une pluralité d'implants à partir d'une cornée humaine ou animale préalablement prélevée, le procédé comprenant les étapes suivantes :

   - déposer (200) la cornée dans un dispositif de maintien incluant des première et deuxième plaques (1, 2) transparentes au faisceau L.A.S.E.R. émis par une source L.A.S.E.R., la cornée étant positionnée entre les première et deuxième plaques (1, 2) pour appliquer une contrainte mécanique sur les faces antérieure et postérieure de la cornée,
   - découper (300), en utilisant le faisceau L.A.S.E.R., la cornée contenue dans le dispositif de maintien pour obtenir une cornée découpée, l'étape de découpe (300) comprenant la génération de bulles de gaz pour former des contours de la pluralité d'implants,
- détacher (400) chaque implant de la cornée découpée,
- dé-cellulariser (500) chaque implant détaché pour obtenir des implants dé-cellularisés,
- lyophiliser (600) chaque implant dé-cellularisé pour obtenir des implants lyophilisés,
- stériliser (700) chaque implant lyophilisé pour obtenir des implants stérilisés,
- emballer (800) chaque implant stérilisé pour obtenir des implants emballés.

2. Procédé de découpe selon la revendication 1, *dans lequel* lesdits implants incluent :

- au moins un implant de renfort incluant une lame (61) circulaire à faces parallèles, et/ou
- au moins un implant de comblement (62, 63) incluant :

o une lame circulaire et un téton s'étendant en saillie de l'une des faces de la lame circulaire, ou
o une rondelle de forme annulaire, et/ou

- au moins un implant (bi)convexe (64), et/ou
- au moins un implant (bi)concave (65), et/ou
- au moins un implant servant de support de culture cellulaire (66) et incluant une lamelle circulaire à faces parallèles et d'épaisseur inférieure au implant de renfort.

3. Procédé de découpe selon l'une quelconque des revendications 1 ou 2, *dans lequel* l'étape consistant à déposer (200) la cornée dans un dispositif de maintien comprend les sous-étapes consistant à :

- positionner le dispositif de maintien sur le chemin optique du faisceau L.A.S.E.R. généré par la source L.A.S.E.R. et orienter le dispositif de maintien de sorte que la première plaque (1) soit plus proche de la source L.A.S.E.R. que la deuxième plaque (2),
- émettre le faisceau L.A.S.E.R. généré par la source L.A.S.E.R. au travers de la première plaque (1) pour former des bulles de gaz dans la moitié de l'épaisseur de la cornée la plus proche de la première plaque (1),
- retourner le dispositif de maintien de sorte que la deuxième plaque (2) soit plus proche de la source L.A.S.E.R. que la première plaque (1),
- émettre le faisceau L.A.S.E.R. généré par la source L.A.S.E.R. au travers de la deuxième plaque (2) pour former des bulles de gaz dans la moitié de l'épaisseur de la cornée la plus proche de la deuxième plaque (2).

4. Procédé selon l'une quelconque des revendications 1 à 3, *lequel* comprend en outre une étape de détermination (100) de zones de coupe dans la cornée, lesdites zones de coupe correspondant à des surfaces de la cornée au niveau desquelles des bulles de gaz doivent être formées lors de l'étape de découpe (300).

5. Procédé de découpe selon la revendication 4, *dans lequel* l'étape de détermination (100) comprend les sous-étapes suivantes :

- acquisition (110) d'une image de la cornée,
- estimation (120) de l'épaisseur et du diamètre de la cornée,
- détermination (130) de paramètres de réglage du dispositif de maintien pour appliquer la contrainte mécanique sur la cornée.

6. Procédé de découpe selon l'une des revendications 4 ou 5, *dans lequel* l'étape de détermination (100) comprend les sous-étapes suivantes :

- détermination (140) des types d'implant souhaités et de leurs tailles associées,
- calcul (150) des positions et formes des zones de coupe à réaliser en fonction des types et tailles des implants souhaités, et
- génération d'un plan de coupe pour minimiser les pertes en tissu cornéen.

7. Procédé de découpe selon la revendication 6, *dans lequel* l'étape de détermination (100) comprend la sous-étape suivante :

- affichage (160) du plan de coupe, ledit plan de coupe illustrant la cornée et les zones de coupe.

8. Procédé de découpe selon l'une quelconque des revendications 1 à 7, *dans lequel* l'étape de dé-cellularisation (500) comprend les sous-étapes suivantes consistant à :

- immerger la cornée reçue dans un liquide de dé-cellularisation, et
- rincer la cornée avec un liquide de rinçage tel qu'une solution sérum physiologique.

9. Procédé de découpe selon l'une des revendications précédentes, *dans lequel* l'étape de dépôt de la cornée comprend une sous-étape consistant à appliquer (210) un matériau favorisant la fixation des protéines - tel que la glutaraldéhyde - sur la cornée.

10. Kit chirurgical pour le traitement d'une pathologie oculaire, *caractérisé en ce que* le kit comprend au moins un implant obtenu en mettant en oeuvre le procédé selon l'une des revendications 1 à 9.

## Patentansprüche

1. Verfahren zum Herstellen einer Vielzahl von Implantaten aus einer zuvor entnommenen menschlichen oder tierischen Hornhaut, wobei das Verfahren folgende Schritte umfasst:

   - Ablegen (200) der Hornhaut in eine Haltevorrichtung, die erste und zweite Scheiben (1, 2) umfasst, die für den Laserstrahl, der von einer Laserquelle emittiert wird, durchlässig sind, wobei die Hornhaut zwischen der ersten und der zweiten Scheibe (1, 2) positioniert wird, um eine mechanische Beanspruchung auf die vordere und die hintere Seite der Hornhaut auszuüben,
   - Schneiden (300), unter Verwendung des Laserstrahls, der in der Haltevorrichtung enthaltenen Hornhaut, um eine geschnittene Hornhaut zu erzielen, wobei der Schritt des Schneidens (300) das Generieren von Gasblasen umfasst, um die Umrisse der Vielzahl von Implantaten zu bilden,
   - Abtrennen (400) jedes Implantats der geschnittenen Hornhaut,
   - Dezellularisieren (500) jedes abgetrennten Implantats, um dezellularisierte Implantate zu erzielen,
   - Lyophilisieren (600) jedes dezellularisierten Implantats, um lyophilisierte Implantate zu erzielen,
   - Sterilisieren (700) jedes lyophilisierten Implantats, um sterilisierte Implantate zu erzielen,
   - Verpacken (800) jedes sterilisierten Implantats, um verpackte Implantate zu erzielen.

2. Schneidverfahren nach Anspruch 1, wobei die Implantate umfassen:

   - mindestens ein Verstärkungsimplantat, das ein kreisförmiges Plättchen (61) mit parallelen Seiten umfasst, und/oder
   - mindestens ein Ausfüllimplantat (62, 63), umfassend:

      o ein kreisförmiges Plättchen und einen Noppen, der sich von einer der Seiten des kreisförmigen Plättchens abstehend erstreckt, oder
      o eine ringförmige Unterlegscheibe, und/oder

   - mindestens ein (bi)konvexes Implantat (64), und/oder
   - mindestens ein (bi)konkaves Implantat (65), und/oder
   - mindestens ein Implantat, das als Zellkulturträger (66) dient und eine kreisförmige Lamelle mit parallelen Seiten und einer geringeren Dicke als das Verstärkungsimplantat umfasst.

3. Schneidverfahren nach einem der Ansprüche 1 oder 2, wobei der Schritt des Ablegens (200) der Hornhaut in einer Haltevorrichtung folgende Teilschritte umfasst:

   - Positionieren der Haltevorrichtung auf dem Lichtweg des Laserstrahls, der von der Laserquelle generiert wird, und Ausrichten der Haltevorrichtung, so dass die erste Scheibe (1) näher als die zweite Scheibe (2) an der Laserquelle ist,
   - Emittieren des Laserstrahls, der von der Laserquelle generiert wird, durch die erste Scheibe (1) hindurch, um Gasblasen in der Hälfte der Dicke der Hornhaut zu bilden, die der ersten Platte (1) am nächsten ist,
   - Umdrehen der Haltevorrichtung, so dass die zweite Scheibe (2) näher als die erste Scheibe (1) an der Laserquelle ist,
   - Emittieren des Laserstrahls, der von der Laserquelle generiert wird, durch die zweite Scheibe (2) hindurch, um Gasblasen in der Mitte der Dicke der Hornhaut zu bilden, die der zweiten Platte (2) am nächsten ist.

4. Verfahren nach einem der Ansprüche 1 bis 3, ferner umfassend einen Schritt des Bestimmens (100) von Schnittbereichen in der Hornhaut, wobei die Schnittbereiche Oberflächen der Hornhaut entsprechen, an denen sich Gasblasen beim Schritt des Schneidens (300) bilden sollen.

5. Schneidverfahren nach Anspruch 4, wobei der Schritt des Bestimmens (100) folgende Teilschritte umfasst:

   - Erfassen (110) eines Bildes der Hornhaut,
   - Schätzen (120) der Dicke und des Durchmessers der Hornhaut,
   - Bestimmen (130) von Parametern zum Einstellen der Haltevorrichtung, um die mechanische Beanspruchung an die Hornhaut anzulegen.

6. Schneidverfahren nach einem der Ansprüche 4 oder 5, wobei der Schritt des Bestimmens (100) folgende Teilschritte umfasst:

   - Bestimmen (140) der gewünschten Implantatarten und ihrer dazugehörigen Größen,
   - Berechnen (150) der Positionen und Formen der Schnittbereiche, die je nach den Arten und Größen der gewünschten Implantate auszubilden sind, und

- Generieren einer Schnittebene, um die Verluste von Hornhautgewebe zu minimieren.

7. Schneidverfahren nach Anspruch 6, wobei der Schritt des Bestimmens (100) den folgenden Teilschritt umfasst:

- Anzeigen (160) der Schnittebene, wobei die Schnittebene die Hornhaut und die Schnittbereiche darstellt.

8. Schneidverfahren nach einem der Ansprüche 1 bis 7, wobei der Schritt des Dezellularisierens (500) die folgenden Teilschritte umfasst:

- Eintauchen der empfangenen Hornhaut in eine Dezellularisierungsflüssigkeit, und
- Spülen der Hornhaut mit einer Spülflüssigkeit, wie etwa mit einer Kochsalzlösung.

9. Schneidverfahren nach einem der vorhergehenden Ansprüche, wobei der Schritt des Ablegens der Hornhaut einen Teilschritt des Auftragens (210) eines Materials umfasst, das die Fixierung der Proteine - wie etwa von Glutaraldehyd - an der Hornhaut begünstigt.

10. Chirurgisches Set für die Behandlung einer Augenerkrankung, **dadurch gekennzeichnet, dass** das Set mindestens ein Implantat umfasst, das durch die Umsetzung des Verfahrens nach einem der Ansprüche 1 bis 9 erzielt wird.

**Claims**

1. A method for producing a plurality of implants from a previously removed human or animal cornea, the method comprising the following steps:

- depositing (200) the cornea in a holding device including first and second plates (1, 2) transparent to the laser beam emitted by a laser source, the cornea being positioned between the first and second plates (1, 2) to apply mechanical stress to the anterior and posterior faces of the cornea,
- cutting (300), using the laser beam, the cornea contained in the holding device to obtain a cut cornea, the cutting step (300) comprising generating gas bubbles to form contours of the plurality of implants,
- detaching (400) each implant from the cut cornea,
- decellularizing (500) each detached implant to obtain decellularized implants,
- lyophilizing (600) each decellularized implant to obtain lyophilized implants,

- sterilizing (700) each lyophilized implant to obtain sterilized implants,
- packaging (800) each sterilized implant to obtain packaged implants.

2. The cutting method as claimed in claim 1, *wherein* said implants include:

- at least one reinforcing implant including a circular blade (61) with parallel faces, and/or
- at least one filling implant (62, 63) including:

  ◦ a circular blade and a nipple protruding from one of the faces of the circular blade, or
  ◦ a ring-shaped washer, and/or

- at least one (bi)convex implant (64), and/or
- at least one (bi)concave implant (65), and/or
- at least one implant serving as a cell culture support (66) and including a circular lamella with parallel faces and a thickness less than that of the reinforcing implant.

3. The cutting method as claimed in any one of claims 1 or 2, *wherein* the step of depositing (200) the cornea in a holding device comprises the substeps consisting in:

- positioning the holding device in the optical path of the laser beam generated by the laser source and orienting the holding device so that the first plate (1) is closer to the laser source than the second plate (2),
- emitting the laser beam generated by the laser source through the first plate (1) to form gas bubbles in the half-thickness of the cornea nearest the first plate (1),
- turn the holding device so that the second plate (2) is closer to the laser source than the first plate (1),
- emitting the laser beam generated by the laser source through the second plate (2) to form gas bubbles in the half-thickness of the cornea nearest the second plate (2).

4. The method as claimed in any one of claims 1 to 3, *which* further comprises a step of determining (100) cutting areas in the cornea, said cutting areas corresponding to surfaces of the cornea at which gas bubbles are to be formed during the cutting step (300).

5. The cutting method as claimed in claim 4, *wherein* the determination step (100) comprises the following substeps:

- acquisition (110) of an image of the cornea,
- estimation (120) of the thickness and diameter of the cornea,
- determination (130) of setting parameters of the holding device to apply the mechanical stress to the cornea.

6. The cutting method as claimed in one of claims 4 or 5, *wherein* the determination step (100) comprises the following substeps:

   - determining (140) the desired implant types and their associated sizes,
   - calculating (150) the positions and shapes of the cutting areas to be made according to the types and sizes of the desired implants, and
   - generating a cutting plane to minimize corneal tissue losses.

7. The cutting method as claimed in claim 6, *wherein* the determination step (100) comprises the following substep:

   - displaying (160) the cutting plane, said cutting plane illustrating the cornea and the cutting areas.

8. The cutting method as claimed in any one of claims 1 to 7, *wherein* the decellularization step (500) comprises the following substeps consisting in:

   - immersing the received cornea in a decellularization fluid, and
   - rinsing the cornea with a rinsing liquid such as saline solution.

9. The cutting method as claimed in any one of the preceding claims, *wherein* the step of depositing the cornea comprises a substep consisting in applying (210) a protein binding-promoting material-such as glutaraldehyde-to the cornea.

10. A surgical kit for treating ocular pathology, *characterized in that* the kit comprises at least one implant obtained by implementing the method as claimed in one of claims 1 to 9.

EP 4 003 248 B1

**FIG.1**

16

100

**DETERMINATION**

110

| ACQUERIR IMAGE CORNEE |

120

| ESTIMER EPAISSEUR ET DIAMETRE |

130

| DETERMINER AJUSTEMENT |

140

| DETERMINER TYPE IMPLANTS |

150

| CALCULER ZONE DE COUPE |

160

| AFFICHER PLAN DE COUPE |

**FIG.2**

6

61

62

63

64

65

66

**FIG.3**

67

**FIG.4**

68

**FIG.5**

FIG.6

**FIG.7**

**FIG.8**

**FIG.9**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2017319329 A
- US 20190038399 A
- FR 1870835